(19) Europäisches Patentamt
European Patent Office
Office européen des brevets

(11) EP 2 305 718 B1

(12) **EUROPÄISCHE PATENTSCHRIFT**

(45) Veröffentlichungstag und Bekanntmachung des Hinweises auf die Patenterteilung:
**17.01.2018 Patentblatt 2018/03**

(21) Anmeldenummer: **10182067.8**

(22) Anmeldetag: **23.03.2006**

(51) Int Cl.:
***C08F 2/04*** (2006.01)
***F26B 17/00*** (2006.01)
***C08F 6/00*** (2006.01)
***C08J 3/24*** (2006.01)
***F26B 17/04*** (2006.01)
*C08F 20/06* (2006.01)
***C08J 3/075*** (2006.01)
***C08F 6/10*** (2006.01)
***A61L 15/60*** (2006.01)
***F26B 21/06*** (2006.01)
***B01J 20/30*** (2006.01)

(54) **Verfahren zur Herstellung wasserabsorbierender Polymere**

Method for producing water-absorbent polymers

Procédé de fabrication de polymères absorbant de l'eau

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **24.03.2005 DE 102005014291**

(43) Veröffentlichungstag der Anmeldung:
**06.04.2011 Patentblatt 2011/14**

(62) Dokumentnummer(n) der früheren Anmeldung(en) nach Art. 76 EPÜ:
**06725287.4 / 1 863 852**

(73) Patentinhaber: **BASF SE**
**67056 Ludwigshafen am Rhein (DE)**

(72) Erfinder:
- **Weismantel, Matthias**
  **63637, Jossgrund (DE)**
- **Bruhns, Stefan**
  **68165, Mannheim (DE)**
- **van Esbroeck, Dominicus**
  **210019 Nanjing (CN)**

(74) Vertreter: **BASF IP Association**
**BASF SE**
**G-FLP-C006**
**67056 Ludwigshafen (DE)**

(56) Entgegenhaltungen:
**DE-A1- 19 846 413**

- **BUCHHOLZ F L ET AL: "Modern superabsorbent polymer technology", 31. Dezember 1998 (1998-12-31), 19981231, XP002619384, * Seite 88 - Seite 91 ***

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 305 718 B1

## Beschreibung

Die vorliegende Erfindung betrifft ein Verfahren zur Herstellung wasserabsorbierender Polymere mit niedrigem Trocknungsqualitätsindex durch Polymerisation einer Monomerlösung und Trocknung des erhaltenen Hydrogels mittels eines erwärmten Gasstromes.

Wasserabsorbierende Polymere sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate. Solche Polymere werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Die Herstellung der wasserabsorbierenden Polymere wird beispielsweise in der Monographie "Modern Superabsorbent Polymer Technology", F.L. Buchholz und A.T. Graham, Wiley-VCH, 1998, oder in Ullmann's Encyclopedia of Industrial Chemistry, 6. Auflage, Band 35, Seiten 73 bis 103, beschrieben.

Üblicherweise wird nach der Polymerisation ein wässriges Polymergel erhalten, das getrocknet werden muss. Die Trocknung des Polymergels wird ebenfalls in der Monographie "Modern Superabsorbent Polymer Technology", F.L. Buchholz und A.T. Graham, Wiley-VCH, 1998, Seiten 87 bis 93, offenbart.

DE 198 46 413 A1 beschreibt ein Verfahren zur Trocknung eines wäßrigen Polymergels, wobei das Polymergel vor der Trocknung unter definierten Bedingungen zerkleinert wird.

Den Trocknungsverfahren ist gemein, dass aufgrund der breiten Gelgrößenverteilung des zu trocknenden Polymerguts eine vollständige Trocknung aller Hydrogelpartikel nur unter solchen Bedingungen erfolgt, unter denen der Großteil der Hydrogelpartikel bereits übertrocknet ist. Diese Trocknungsbedingungen stellen aber eine unwirtschaftliche Ausnutzung der Trocknerkapazität dar. Bei einer wirtschaftlich optimierten Ausnutzung der Trocknerkapazität sind die Trocknungsbedingungen jedoch derart, dass der Großteil der Hydrogelpartikel bereits trocken ist, während ein kleinerer Teil der Hydrogelpartikel noch feucht ist. Feuchte Hydrogelpartikel sind gummielastisch und neigen zu Verklebungen, so dass sie zu erheblichen Störungen bei dem sich anschließenden Mahl- und Siebprozess des Trocknungsgutes führen, welche unerwünscht sind. Es besteht daher die Notwendigkeit, die feuchten, gummielastischen Hydrogelteilchen von den spröden, teilweise übertrockneten Hydrogelteilchen vor der Mahlung abzutrennen. In der Praxis werden daher Trocknungsbedingungen gewählt, die einen Kompromiss zwischen Ausnutzung der Trocknerkapazität und Verarbeitbarkeit des Trocknungsgutes darstellen.

Aufgabe der vorliegenden Erfindung war die Bereitstellung eines verbesserten Verfahrens zur Herstellung wasserabsorbierender Polymerpartikel, insbesondere eine verbesserte Trocknung der während des Verfahrens anfallenden wässrigen Polymergele.

Das Trocknungsverfahren sollte einerseits wirtschaftlich sein und bereits nach kurzen Verweilzeiten zu einem Produkt mit niedrigem Wassergehalt führen, andererseits sollte das Trocknungsverfahren sehr schonend sein, so dass die Produktqualität durch die Trocknung nur wenig verändert wird.

Gelöst wurde die Aufgabe durch Verfahren zur Herstellung wasserabsorbierender Polymere durch Polymerisation einer Monomerlösung, enthaltend mindestens eine ethylenisch ungesättigte Carbonsäure und mindestens einen Vernetzer, und Trocknung des erhaltenen Hydrogels mittels eines erwärmten Gasstromes, dadurch gekennzeichnet, dass

- optional die Trocknung in mindestens zwei Temperaturzonen durchgeführt wird, wobei die Gaseingangstemperaturen der mindestens zwei Temperaturzonen die Bedingung $T_n$ ungleich $T_{n+a}$ erfüllen, wobei die Indizes n und a jeweils eine ganze Zahl größer 0, vorzugsweise eine ganze Zahl von 1 bis 20, besonders bevorzugt eine ganze Zahl von 1 bis 10, ganz besonders bevorzugt eine ganze Zahl von 1 bis 5, bedeuten, und

- optional der Gasstrom das Hydrogel im vorderen Abschnitt eines Bandtrockners von unten und im hinteren Abschnitt des Bandtrockners von oben anströmt, wobei die Strömungsumkehr bei einem Wassergehalt des Hydrogels von 15 bis 45 Gew.-% stattfindet, und

- die Hydrogelschicht in einem Bandtrockner zumindest teilweise von unten angeströmt wird, wobei die Gasgeschwindigkeit von 10 bis 20% der Gasgeschwindigkeit beträgt, die notwendig ist um das Hydrogel vom Band zu lösen.

Die Temperatur des erwärmten Gasstroms beträgt vorzugsweise mindestens 50°C, besonders bevorzugt mindestens 100°C, ganz besonders bevorzugt mindestens 150°C, und vorzugsweise bis zu 250°C, besonders bevorzugt bis zu 220°C, ganz besonders bevorzugt bis zu 200°C.

Die Indizes geben die zeitliche Abfolge der Temperaturzonen an, die das zu trocknende Gut in aufsteigender Abfolge durchläuft, wobei Temperaturzonen mit höheren Indizes später durchlaufen werden. Eine Temperaturzone ist

ein Bereich, in dem die Gaseingangstemperatur unabhängig eingestellt werden kann.

Der vordere Abschnitt besteht aus Temperaturzonen mit niedrigeren Indizes, der hintere Abschnitt besteht aus Temperaturzonen mit höheren Indizes. Damit durchläuft das zu trocknende Gut zuerst den vorderen Abschnitt.

Der Wassergehalt wird gemäß der von der EDANA (European Disposables and Nonwovens Association) empfohlenen Testmethode Nr. 430.2-02 "Moisture content" bestimmt.

Die Gas- bzw. Luftgeschwindigkeit, bei der sich die Hydrogelschicht vom Band ablöst (Wirbelpunkt), kann experimentell bestimmt oder gemäß $v_{max} = \sqrt{\frac{\rho_B \times g \times \Delta h}{c_D}}$ berechnet werden, wobei $v_{max}$ die maximale Gas- bzw. Luftgeschwindigkeit ist, bei der sich das Hydrogel vom Band ablöst $\rho_B$ das Schüttgewicht des Hydrogels, g die Gravitationskonstante, $\Delta h$ der Druckverlust über die Hydrogelschicht und $c_D$ der Gas- bzw. Luftwiderstandsbeiwert ist. Am Wirbelpunkt heben sich die auf die Hydrogelschicht wirkende Schwerkraft und der Gas- bzw Luftwiderstand auf. Der Wirbelpunkt markiert die Grenze zwischen Festbett und Wirbelbett. Das Schüttgewicht des Hydrogels ist der Quotient aus Gewicht und Schüttvolumen des Hydrogels auf dem Band. Das Schüttvolumen des Hydrogels schließt neben dem Hydrogel noch die im Hydrogel enthaltenen Hohlräume mit ein.

Vorzugsweise erfüllen die mindestens zwei Gaseingangstemperaturen die Bedingung $T_n$ größer $T_{n+a}$.

Bevorzugt wird die Trocknung in mindestens drei Temperaturzonen durchgeführt, wobei die Gaseingangstemperaturen die Bedingungen $T_n$ ungleich $T_{n+a}$, vorzugsweise $T_n$ größer $T_{n+a}$. und $T_{n+a}$ kleiner $T_{n+b}$ erfüllen, wobei der Index b eine ganze Zahl größer a, vorzugsweise eine ganze Zahl von (a+1) bis (a+20), besonders bevorzugt eine ganze Zahl von (a+1) bis (a+10), ganz besonders bevorzugt eine ganze Zahl von (a+1) bis (a+5), bedeutet, wobei vorzugsweise gilt $T_n$ größer $T_{n+b}$.

In einer besonders bevorzugten Ausführungsform der vorliegenden Erfindung erfüllen die Gaseingangstemperaturen in mindestens zwei der a Temperaturzonen $T_n$ bis $T_{n+a-1}$ die Bedingung $T_{n+r}$ größer $T_{n+s}$, wobei der Index a eine ganze Zahl größer 1, der Index r eine ganze Zahl von 0 bis (a-2) und der Index s eine ganze Zahl von (r+1) bis (a-1) bedeuten.

In einer weiteren besonders bevorzugten Ausführungsform der vorliegenden Erfindung erfüllen die Gaseingangstemperaturen in mindestens zwei der (c-b) Temperaturzonen $T_{n+b}$ bis $T_{n+c-1}$ die Bedingung $T_{n+u}$ größer $T_{n+v}$, wobei der Index c eine ganze Zahl größer (b+1), vorzugsweise eine ganze Zahl von (b+1) bis (b+20), besonders bevorzugt eine ganze Zahl von (b+1) bis (b+10), ganz besonders bevorzugt eine ganze Zahl von (b+1) bis (b+5), der Index u eine ganze Zahl von b bis (c-2) und der Index v eine ganze Zahl von (u+1) bis (c-1) bedeuten.

In einer ganz besonders bevorzugten Ausführungsform der vorliegenden Erfindung erfüllen die Gaseingangstemperaturen in mindestens drei der (c-b) Temperaturzonen $T_{n+b}$ bis $T_{n+c-1}$ die Bedingung $T_{n+u}$ größer $T_{n+v}$ größer $T_{n+w}$, wobei der Index c eine ganze Zahl größer (b+2), vorzugsweise eine ganze Zahl von (b+2) bis (b+20), besonders bevorzugt eine ganze Zahl von (b+2) bis (b+10), ganz besonders bevorzugt eine ganze Zahl von (b+2) bis (b+5), der Index u eine ganze Zahl von b bis (c-3), der Index v eine ganze Zahl von (u+1) bis (c-2) und der Index w eine ganze Zahl von (v+1) bis (c-1) bedeuten.

Ganz besonders bevorzugt ist ein Verfahren zur Trocknung von wässrigen Hydrogelen in mindestens sechs Temperaturzonen, wobei a mindestens 2, b mindestens 3 und c mindestens 6 beträgt. Vorzugsweise werden die Gaseingangstemperaturen so eingeselft, dass gilt $T_n$ größer $T_{n+b}$, $T_{n+1}$ größer $T_{n+b+1}$ sowie $T_{n+1}$ nicht kleiner $T_{n+b}$.

Die optimale und damit bevorzugte Temperaturverteilung läßt sich auch als Welle, bestehend aus zwei Wellenbergen und einem Wellental, darstellen. Dabei ist der erste Wellenberg $T_n$, der zweite Wellenberg $T_{n+b}$ und das Wellental dazwischen $T_{n+a}$, wobei der erste Wellenberg höher ist als der zweite.

Der Temperaturdifferenz der Gaseingangstemperaturen beträgt, sofern eine Temperaturdifferenz gefordert, üblicherweise mindesten 0,5°C, vorzugsweise mindestens 1°C, besonders bevorzugt mindestens 5°C, ganz besonders bevorzugt mindestens 10°C, und üblicherweise bis zu 50°C, vorzugsweise bis zu 40°C, besonders bevorzugt bis zu 30°C, ganz besonders bevorzugt bis zu 20°C.

Die Geschwindigkeit des die Hydrogelschicht anströmenden Gasstromes beträgt vorzugsweise mindestens 0,5 m/s, besonders bevorzugt mindestens 0,8 m/s, ganz besonders bevorzugt mindestens 1 m/s, und vorzugsweise bis zu 5 m/s, besonders bevorzugt bis zu 3 m/s, ganz besonders bevorzugt bis zu 2 m/s.

Das zu verwendende Gas unterliegt keiner Beschränkung. Zur Trocknung können Luft, Stickstoff oder andere unter den Trockenbedingungen inerte Gase eingesetzt werden. Luft ist bevorzugt.

Der das Hydrogel anströmende Gasstrom kann Wasserdampf enthalten. Der Wasserdampfanteil sollte aber einen Wert, der einem Taupunkt von vorzugsweise höchstens 50°C, besonders bevorzugt höchstens 40°C, ganz besonders bevorzugt höchstens 30°C, entspricht, nicht übersteigen.

Die Gaseingangstemperaturen $T_n$ bis $T_{n+a-1}$ betragen vorzugsweise höchstens 200°C, besonders bevorzugt von 175 bis 180°C.

Die Gaseingangstemperaturen $T_{n+a}$ bis $T_{n+b-1}$ betragen vorzugsweise mindestens 150°C, besonders bevorzugt

mindestens 155°C, ganz besonders bevorzugt von 155 bis 160°C.

Die Gaseingangstemperaturen $T_{n+b}$ bis $T_{n+c-1}$ betragen vorzugsweise höchstens 185°C, besonders bevorzugt höchstens 180°C, ganz besonders bevorzugt von 170 bis 175°C.

Die Verweilzeit bei der Trocknung beträgt vorzugsweise mindestens 10 Minuten, besonders bevorzugt mindestens 20 Minuten, ganz besonders bevorzugt mindestens 30 Minuten, und vorzugsweise bis zu 120 Minuten, besonders bevorzugt bis zu 90 Minuten, ganz besonders bevorzugt bis zu 60 Minuten.

Die relative Verweilzeit beträgt für die Summe der Verweilzeiten der Temperaturzonen mit den Gaseingangstemperaturen $T_n$ bis $T_{n+a-1}$ vorzugsweise mindestens 10%, besonders bevorzugt mindestens 15%, vorzugsweise bis zu 25%, ganz besonders bevorzugt bis zu 20%, ganz besonders bevorzugt 18%, für die Summe der Verweilzeiten der Temperaturzonen mit den Gaseingangstemperaturen $T_{n+a}$ bis $T_{n+b-1}$ vorzugsweise mindestens 5%, besonders bevorzugt mindestens 10%, vorzugsweise bis zu 20%, ganz besonders bevorzugt bis zu 16%, ganz besonders bevorzugt 14%, und für die Summe der Verweilzeiten der Temperaturzonen mit den Gaseingangstemperaturen $T_{n+b}$ bis $T_{n+c-1}$ vorzugsweise mindestens 80%, besonders bevorzugt mindestens 70%, vorzugsweise bis zu 40%, ganz besonders bevorzugt bis zu 60%, ganz besonders bevorzugt 68%, jeweils bezogen auf die Gesamtverweilzeit auf dem Trockner.

Die relative Verweilzeit in den a einzelnen Temperaturzonen $T_n$ bis $T_{n+a-1}$ wird vorzugsweise so eingestellt, dass die relativen Verweilzeiten gleich sind.

Die relative Verweilzeit in den (b-a) einzelnen Temperaturzonen $T_{n+a}$ bis $T_{n+b-1}$ wird vorzugsweise so eingestellt, dass die relativen Verweilzeiten gleich sind.

Die relative Verweilzeit in den (c-b) einzelnen Temperaturzonen $T_{n+b}$ bis $T_{n+c-1}$ wird vorzugsweise so eingestellt, dass die relativen Verweilzeiten gleich sind.

Wird das zu trocknende Hydrogel im vorderen Abschnitt des Bandtrockners von unten und im hinteren Abschnitt des Bandtrockners von oben von dem Gasstrom angeströmt, so beträgt der Wassergehalt des Hydrogels bei der Strömungsumkehr vorzugsweise mindestens 20 Gew.-%, bevorzugt mindestens 24 Gew.-%, besonders bevorzugt mindestens 26 Gew.-%, ganz besonders bevorzugt mindestens 28 Gew.-%, und vorzugsweise höchstens 40 Gew.-%, bevorzugt höchstens 34 Gew.-%, besonders bevorzugt höchstens 32 Gew.-%, ganz besonders bevorzugt höchstens 30 Gew.-%. Die Trocknung wird vorzugsweise so betrieben, dass die Strömungsumkehr zwischen den Temperaturzonen $T_{n+b-1}$ und $T_{n+b}$ eintritt.

Vorzugsweise ist die Gasgeschwindigkeit nach der Strömungsumkehr erhöht, vorzugsweise um mindestens 10%, besonders bevorzugt um mindestens 30%, ganz besonders um mindestens 40%, und vorzugsweise um bis zu 100%, besonders bevorzugt um bis zu 80%, ganz besoders um bis zu 60%.

Der Wassergehalt des zu trocknenden Polymergels beträgt vorzugsweise mindestens 30 Gew.-%, besonders bevorzugt mindestens 40 Gew.-%, ganz besonders bevorzugt mindestens 50 Gew.-%, und vorzugsweise bis zu 70 Gew-%, besonders bevorzugt bis zu 65 Gew.-%, ganz besonders bevorzugt bis zu 60 Gew.-%.

Der Wassergehalt des getrockneten Polymergels beträgt vorzugsweise mindestens 2 Gew.-%, besonders bevorzugt mindestens 3 Gew.-%, ganz besonders bevorzugt mindestens 5 Gew.-%, und vorzugsweise bis zu 10 Gew.-%, besonders bevorzugt bis zu 9 Gew.-%, ganz besonders bevorzugt bis zu 8 Gew.-%.

Die Trocknung wird vorzugsweise bei einem Druck durchgeführt, der gegenüber dem Atmosphärendruck vermindert ist, vorzugsweise um mindestens 0,5 mbar, besonders bevorzugt um mindestens 2 mbar, ganz besonders bevorzugt um mindestens 10 mbar.

Der gegenüber dem Atmosphärendruck verminderte Druck im Trockner bewirkt eine günstigere Gasströmung im Trockner und damit eine gleichmäßigere Trocknung.

Das für die vorliegende Erfindung bevorzugte Verfahren ist ein Förderbandverfahren (Bandtrockner). Der Bandtrockner ist ein konvektives Trocknungssystem für die besonders schonende Behandlung von durchlüftbaren Produkten. Das zu trocknende Produkt wird auf ein endloses, gasdurchlässiges Förderband gegeben und mittels eines erwärmten Gasstromes, vorzugsweise Luft, angeströmt.

Das Trocknungsgas kann im Kreis geführt werden, um beim mehrfachen Durchgang durch die Produktschicht eine möglichst hohe Aufsättigung zu erfahren. Ein gewisser Anteil des Trockengases, vorzugsweise mindestens 10%, besonders bevorzugt mindestens 15%, ganz besonders bevorzugt mindestens 20%, und vorzugsweise bis zu 50%, besonders bevorzugt bis zu 40%, ganz besonders bevorzugt bis zu 30%, der Gasmenge pro Durchgang, verlässt den Trockner als hoch aufgesättigter Brüden und führt die aus dem Produkt verdampfte Wassermenge ab.

Die Größe und Ausführung der Trockner richtet sich nach dem zu verarbeitenden Produkt, der Produktionskapazität und der Trocknungsaufgabe.

Der Bandtrockner kann als Einband-, Mehrband-, Mehrstufen-, oder Mehretagensystem ausgestattet werden. Bevorzugt ist für die vorliegende Erfindung der Betrieb eines Bandtrockners mit mindestens einem Band. Ganz besonders bevorzugt sind Einbandtrockner. Um die Bandtrocknung verfahrenstechnisch optimal durchzuführen, werden die Trocknungseigenschaften der wasserabsorbierenden Polymere in Abhängigkeit von den gewählten Prozessparametern individuell ermittelt. Die Lochgröße und Maschenweite des Bandes wird dem Produkt angepaßt. Auch bestimmte Oberflächenveredelungen, wie Elektropolieren oder Teflonisieren, sind möglich.

Zur optimalen Produktförderung können alle, dem Fachmann bekannten kettengeführten und kettenlosen Bandsysteme eingesetzt werden, wie beispielsweise Plattenbänder, Dünnblech- und Endlosplattenbänder, Kunststoff- und Metallgewebebänder.

Zur wirtschaftlichen Trocknung der wasserabsorbierenden Polymere wird die Gasführung im Trockner konsequent auf einen energieeffizienten Betrieb ausgelegt. Es sind verschiedene Gasführungskonzepte möglich, die Vorteile hinsichtlich Trocknungsverhalten und Energieausnutzung aufweisen. Energierückgewinnungssysteme können eingesetzt werden, um Wärme aus dem Abgasstrom zur Vorwärmung des zugeführten Frischgases zu nutzen.

Die Gasführung kann nach folgenden Konzepte erfolgen: im Kreuzstrom von oben / von unten, alternierend, Kreuzgegenstrom oder aber im Kreuzgleichstrom. Die Gasführung im Kreuzgegenstrom ist bevorzugt.

Die Beheizung des Trockners kann direkt oder indirekt über die verschiedenen Heizmedien wie Dampf, Warmwasser, Rauchgase, Thermalöl oder Gas erfolgen.

Der Einbandtrockner zeichnet sich durch eine nur geringe Bauhöhe aus. Er wird eingesetzt zur schonenden Trocknung und wenn Umschüttungen nicht möglich oder erwünscht sind.

Bei geringem Platzangebot und sehr langen Trocknungszeiten bietet sich häufig das Konzept eines Mehrbandtrockners an. Das Produkt wird gleichmäßig auf das oberste Band verteilt und nacheinander an mehrere darunterliegende Bänder weitergegeben. Es ergibt sich der Vorteil, dass das Produkt beim Übergang und Fallen auf die nächste Ebene mehrfach gewendet und homogenisiert wird. Die Umschüttung des Produktes bei der Übergabe von einem auf das nächste Band führt zur Auflösung von Agglomeraten und Schaffung neuer freier Oberflächen für den Wärme- und Stoffübergang.

Mehretagentrockner weisen ähnliche Merkmale wie Mehrbandtrockner auf, allerdings sind die einzelnen Sektionen unabhängig voneinander steuerbar wie bei Einbandtrocknern. Der Mehrstufentrockner besteht aus mehreren hintereinander geschalteten Einbandtrocknern.

Eine wesentliche Voraussetzung zur optimalen Trocknung ist die gleichmäßige Produktaufgabe. Diese kann gestaltet werden durch den Einsatz von schwenkbaren und oszillierenden Verteilbändern, Schwingrinnen oder Schnecken, Vibrationsrinnen oder Schwingförderern.

Das zu trocknende Hydrogel wird vorzugsweise mittels eines Schwenkbandes auf das Band des Bandtrockners aufgebracht. Die Aufgabehöhe, d.h., der vertikale Abstand zwischen Schwenkband und Band, beträgt vorzugsweise mindeste 10 cm, besonders bevorzugt mindestes 20 cm, ganz besonders bevorzugt mindestens 30 cm, vorzugsweise bis zu 200 cm, besonders bevorzugt bis zu 120 cm, ganz besonders bevorzugt bis zu 40 cm.

Die Schichtdicke des zu trocknende Hydrogels auf dem Bandtrockner beträgt vorzugsweise mindestens 2 cm, besonders bevorzugt mindestens 5 cm, ganz besonders bevorzugt mindestens 8 cm, und vorzugsweise höchstens 20 cm, besonders bevorzugt höchstens 15 cm, ganz besonders bevorzugt höchstens 12 cm.

Die Bandgeschwindigkeit des Bandtrockners beträgt vorzugsweise mindestens 0,005 m/s, besonders bevorzugt mindestens 0,01 m/s, ganz besonders bevorzugt mindestens 0,015 m/s, und vorzugsweise bis zu 0,05 m/s, besonders bevorzugt bis zu 0,03 m/s, ganz besonders bevorzugt bis zu 0,025 m/s.

Die Trocknung nach dem Förderband-Verfahren (Bandtrocknung), bei der mit Löcher versehene Horden eines Kreisförderers in einem Tunnel in der oben angegebenen Weise mit Trocknungsgut beladen und das Trocknungsgut während der Förderung durch Durchblasen von Gas/Luft/Gemisch in der oben angegebenen Weise durch die Hordenlöcher getrocknet wird, stellt das wirtschaftlichste Trocknungsverfahren für wasserabsorbierende Polymere dar und ist daher bevorzugt. Die Trocknungsgeschwindigkeit des Trocknungsguts wird bestimmt durch die Verdampfungsleistung, die angibt, wieviel kg Wasser aus dem zu trocknenden Produkt pro Quadratmeter Bandfläche pro Stunde herausgetrocknet werden. Diese Verdampfungsleistung sollte aus wirtschaftlichen Gründen möglichst hoch sein.

Die nach dem erfindungsgemäßen Verfahren zu trocknende Hydrogelstruktur, die gegebenenfalls mit zusätzlichen Reaktanden und/oder wasserabsorbierende Polymerpartikel, die als Unterkorn bei den Klassierschritten abgetrennt wurden, vermischt ist, weist aufgrund ihrer lockeren Anordnung bereits zerteilter Gelkörper eine relativ große Geloberfläche und somit für die Bandtrocknung eine wirtschaftlich vorteilhafte Trocknungsgeschwindigkeit auf. In einer besonders bevorzugten Verfahrensvariante kann die Trocknungsgeschwindigkeit durch Aufgabe eines Trennmittels auf die Hydrogelteilchen noch weiter gesteigert werden. Das Aufbringen der Trennmittel erfolgt hierbei ohne mechanische Belastung der Hydrogelpartikel durch Aufsprühen in dafür geeigneten Geräten, wie beispielsweise Drehrohr, Drais-Mischer, Pflugscharmischern, wie Lödige-Mischer, Peterson-Kelly-Mischer, Kegel-Schnecken-Mischern.

Als Trennmittel eignen sich nichtionische, ionische oder amphotere Tenside mit einem HLB-Wert größer gleich 3 (Definition des HLB-Wertes: siehe W.C. Griffin, J.Soc.Cosmetic Chem. 5 (1954) 249). Bevorzugt sind solche Tenside, die in Wasser löslich oder zumindest dispergierbar sind. Der Einsatz von Tensiden zur Verbesserung der Trocknungseigenschaften von Hydrogelpartikeln bei der Trocknung mit Hilfe von Kontakttrocknern ist bekannt und ausführlich in EP-A-0 785 224 beschrieben. Beispiele zu den als Trennmittel fungierenden Tensiden sind der EP-A-0 785 223 auf Seite 3, Zeile 27, bis Seite 4, Zeile 38, zu entnehmen. Weitere geeignete Trennmittel sind Silicone, ungesättigte Alkohole oder Polyglykole und ihre Derivate. Beispiele der angegebenen Verbindungsklassen sind der DE-A-198 46 413 auf Seite 6, Zeilen 21 bis 42. zu entnehmen.

Bei der im Laufe der Trocknung bewirkten Zerkleinerung von verhältnismäßig großen Stücken zu anschließend feinen Teilchen ist es bevorzugt, die Temperatur des Polymergels bei fortschreitender Trockung so niedrig wie möglich zu halten, um den Wirkungsgrad zu erhöhen und zu verhindern, dass die Stücke oder feinen Teilchen aneinander kleben. Dies kann beispielsweise erreicht werden durch ausreichende Kühlung des gebildeten Polymergels mit anströmender Kaltluft bzw. Umgebungsluft, wobei es durch die Verdampfungskälte zur automatischen Abkühlung des Polymerguts kommt.

Das Polymergel wird auf diese Weise beispielsweise durch Strömungstrocknen auf einem Band getrocknet.

Nach dem erfindungsgemäßen Verfahren ist es möglich wässrige Polymergele zu trocken, so dass der Trocknungsqualitätsindex (TQI) höchstens 8, vorzugsweise höchstens 6, bevorzugt höchstens 4, besonders bevorzugt höchstens 2, ganz besonders bevorzugt höchstens 1, und üblicherweise mindestens 0,01 beträgt.

Besonders vorteilhaft ist das erfindungsgemäße Verfahren, wenn bei der Herstellung der wässrigen Polymergele zumindest teilweise Vernetzer eingesetzt werden, die mindestens zwei ethylenisch ungesättigte Gruppen enthalten und bei denen mindestens zwei ethylenisch ungesättigte Gruppen über mindestens eine Estergruppe miteinander verbunden sind, beispielsweise Ester ungesättigter Mono- oder Polycarbonsäuren von Polyolen, wie Diacrylat oder Triacrylat, beispielsweise Butandiol- oder Ethylenglykoldiacrylat bzw. -methacrylat sowie Trimethylolpropantriacrylat und Allylverbindungen, wie Allyl(meth)acrylat, Triallylcyanurat, Maleinsäurediallylester und Polyallylester.

Die wässrigen Polymergele werden durch Polymerisation einer Monomerlösung, enthaltend

a) mindestens eine ethylenisch ungesättigte Carbonsäure,
b) mindestens einen Vernetzer,
c) gegebenenfalls ein oder mehrere mit dem Monomeren a) copolymerisierbare ethylenisch und/oder allylisch ungesättigte Monomere und
d) gegebenenfalls ein oder mehrere wasserlösliche Polymere, auf die die Monomere a), b) und ggf. c) zumindest teilweise aufgepfropft werden können,

erhalten.

Geeignete Monomere a) sind beispielsweise Acrylsäure, Methacrylsäure, Maleinsäure, Fumarsäure und Itaconsäure. Besonders bevorzugte Monomere sind Acrylsäure und Methacrylsäure. Ganz besonders bevorzugt ist Acrylsäure.

Die Monomere a), insbesondere Acrylsäure, enthalten vorzugsweise bis zu 0,025 Gew.-% eines Hydrochinonhalbethers. Bevorzugte Hydrochinonhalbether sind Hydrochinonmonomethylether (MEHQ) und/oder Tocopherole.

Unter Tocopherol werden Verbindungen der folgenden Formel verstanden

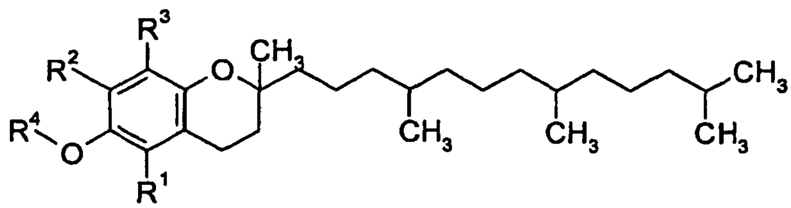

wobei $R^1$ Wasserstoff oder Methyl, $R^2$ Wasserstoff oder Methyl, $R^3$ Wasserstoff oder Methyl und $R^4$ Wasserstoff oder ein Säurerest mit 1 bis 20 Kohlenstoffatomen bedeutet.

Bevorzugte Reste für $R^4$ sind Acetyl, Ascorbyl, Succinyl, Nicotinyl und andere physiologisch verträgliche Carbonsäuren. Die Carbonsäuren können Mono-, Di- oder Tricarbonsäuren sein.

Bevorzugt ist alpha-Tocopherol mit $R^1 = R^2 = R^3$ = Methyl, insbesondere racemisches alpha-Tocopherol. $R^1$ ist besonders bevorzugt Wasserstoff oder Acetyl. Insbesondere bevorzugt ist RRR-alpha-Tocopherol.

Die Monomerlösung enthält bevorzugt höchstens 130 Gew.-ppm, besonders bevorzugt höchstens 70 Gew.-ppm, bevorzugt mindesten 10 Gew.-ppm, besonders bevorzugt mindesten 30 Gew.-ppm, insbesondere um 50 Gew.-ppm, Hydrochinonhalbether, jeweils bezogen auf Acrylsäure, wobei Acrylsäuresalze als Acrylsäure mit berücksichtigt werden. Beispielsweise kann zur Herstellung der Monomerlösung eine Acrylsäure mit einem entsprechenden Gehalt an Hydrochinonhalbether verwendet werden.

Die Vernetzer b) sind Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Allylmethacrylat, Trimethylolpropantriacrylat, Triallylamin, Tetraallyloxyethan, wie in EP-A-0 530 438 beschrieben, Di- und Triacrylate, wie in EP-A-0 547 847, EP-A-0 559 476, EP-A-0 632 068, WO-A-93/21237, WO-A-03/104299, WO-A-03/104300, WO-A-03/104301 und DE-A-103 31 450 beschrieben, gemischte Acrylate, die neben Acrylatgruppen weitere ethylenisch ungesättigte Gruppen enthalten, wie in DE-A-103 31 456 und der

älteren deutschen Anmeldung mit dem Aktenzeichen 10355401.7 beschrieben, oder Vernetzermischungen, wie beispielsweise in DE-A-195 43 368, DE-A-196 46 484, WO-A-90/15830 und WO-A-02/32962 beschrieben.

Geeignete Vernetzer b) sind insbesondere N,N'-Methylenbisacrylamid und N,N'-Methylenbismethacrylamid, Ester ungesättigter Mono- oder Polycarbonsäuren von Polyolen, wie Diacrylat oder Triacrylat, beispielsweise Butandiol- oder Ethylenglykoldiacrylat bzw. -methacrylat sowie Trimethylolpropantriacrylat und Allylverblndungen, wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP-A-0 343 427 beschrieben sind. Weiterhin geeignete Vernetzer b) sind Pentaerythritoldi- Pentaerythritoltri- und Pentaerythritoltetraallylether, Polyethylenglykoldiallylether, Ethylenglykoldiallylether, Glyzerindi- und Glyzerintriallylether, Polyallylether auf Basis Sorbitol, sowie ethoxylierte Varianten davon. Im erfindungsgemäßen Verfahren einsetzbar sind Di(meth)acrylate von Polyethylenglykolen, wobei das eingesetzte Polyethylenglykol ein Molekulargewicht zwischen 300 und 1000 aufweist.

Besonders vorteilhafte Vernetzer b) sind jedoch Di- und Triacrylate des 3- bis 15-fach ethoxylierten Glyzerins, des 3- bis 15-fach ethoxylierten Trimethylolpropans, des 3- bis 15-fach ethoxylierten Trimethylolethans, inbesondere Di- und Triacrylate des 2- bis 6-fach ethoxylierten Glyzerins oder Trimethylolpropans, des 3-fach propoxylierten Glyzerins oder Trimethylolpropans, sowie des 3-fach gemischt ethoxylierten oder propoxylierten Glyzerins oder Trimethylolpropans, des 15-fach ethoxylierten Glyzerins oder Trimethylolpropans, sowie des 40-fach ethoxylierten Glyzerins, Trimethylolethans oder Trimethylolpropans.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine wie sie beispielsweise in WO-A-03/104301 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5- fach ethoxylierten und/oder propoxylierten Glyzerins. Am meisten bevorzugt sind die Triacrylate des 3-bis 5-fach ethoxylierten und/oder propoxylierten Glyzerins. Diese zeichnen sich durch besonders niedrige Restgehalte (typischerweise unter 10 Gew.-ppm) im wasseraborbierenden Polymer aus und die wässrigen Extrakte der damit hergestellten wasserabsorbierenden Polymere weisen eine fast unveränderte Oberflächenspannung (typischerweise mindestens 0,068 N/m) im Vergleich zu Wasser gleicher Temperatur auf.

Mit den Monomeren a) copolymerisierbare ethylenisch ungesättigte Monomere c) sind beispielsweise Acrylamid, Methacrylamid, Crotonsäureamid, Dimethylaminoethylmethacrylat, Dimethylaminoethylacrylat, Dimethylaminopropylacrylat, Diethylaminopropylacrylat, Dimethylaminobutylacrylat, Dimethylaminoethylmethacrylat, Diethylaminoethylmethacrylat, Dimethylaminoneopentylacrylat und Dimethylaminoneopentylmethacrylat.

Als wasserlösliche Polymere d) können Polyvinylalkohol, Polyvinylpyrrolidon, Stärke, Stärkederivate, Polyglykole oder Polyacrylsäuren, vorzugsweise Polyvinylalkohol und Stärke, eingesetzt werden.

Die bevorzugten Polymerisationsinhibitoren benötigen für eine optimale Wirkung gelösten Sauerstoff. Daher können die Polymerisationsinhibitoren vor der Polymerisation durch Inertisierung, d.h. Durchströmen mit einem inerten Gas, vorzugsweise Stickstoff, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, gesenkt.

Die Herstellung eines geeigneten Grundpolymers sowie weitere geeignete hydrophile ethylenisch ungesättigte Monomere d) werden in DE-A-199 41 423, EP-A-0 686 650, WO-A-01/45758 und WO-A-03/104300 beschrieben.

Wasserabsorbierende Polymere werden üblicherweise durch Polymerisation einer wässrigen Monomerlösung und gegebenenfalls einer anschließenden Zerkleinerung des Hydrogels erhalten. Geeignete Herstellverfahren sind in der Literatur beschrieben. Wasserabsorbierende Polymere können beispielsweise erhalten werden durch

- Gelpolymerisation im Batchverfahren bzw. Rohrreaktor und anschließender Zerkleinerung im Fleischwolf, Extruder oder Kneter (EP-A-0 445 619, DE-A-19 846 413)
- Polymerisation im Kneter, wobei durch beispielsweise gegenläufige Rührwellen kontinuierlich zerkleinert wird, (WO-A-01/38402)
- Polymerisation auf dem Band und anschließende Zerkleinerung im Fleischwolf, Extruder oder Kneter (DE-A-38 25 366, US-6,241,928)
- Emulsionspolymerisation, wobei bereits Perlpolymerisate relativ enger Gelgrößenverteilung anfallen (EP-A-0 457 660)
- In-situ Polymerisation einer Gewebeschicht, die zumeist im kontinuierlichen Betrieb zuvor mit wässriger Monomerlösung besprüht und anschließend einer Photopolymerisation unterworfen wurde (WO-A-02/94328, WO-A-02/94329)

Die Umsetzung wird vorzugsweise in einem Kneter, wie beispielsweise in WO-A-01/38402 beschrieben, oder auf einem Bandreaktor, wie beispielsweise in EP-A-0 955 086 beschrieben, durchgeführt.

Die Säuregruppen der erhaltenen Hydrogele sind üblicherweise teilweise neutralisiert, vorzugsweise zu 25 bis

85 mol-%, bevorzugt zu 27 bis 80 mol-%, besonders bevorzugt zu 27 bis 30 mol-% oder 40 bis 75 mol-%, wobei die üblichen Neutralisationsmittel verwendet werden können, vorzugsweise Alkalimetallhydroxide, Alkalimetalloxide, Alkalimetallcarbonate oder Alkalimetallhydrogencarbonate sowie deren Mischungen. Statt Alkalimetallsalzen können auch Ammoniumsalze verwendet werden. Natrium und Kalium als Alkalimetalle besonders bevorzugt sind, ganz besonders bevorzugt jedoch Natriumhydroxid, Natriumcarbonat oder Natriumhydrogencarbonat sowie deren Mischungen. Üblicherweise wird die Neutralisation durch Einmischung des Neutralisationsmittels als wässrige Lösung oder bevorzugt auch als Feststoff erreicht. Beispielsweise kann Natriumhydroxid mit einem Wasseranteil deutlich unter 50 Gew.-% als wachsartige Masse mit einem Schmelzpunkt oberhalb 23°C vorliegen. In diesem Fall ist eine Dosierung als Stückgut oder Schmelze bei erhöhter Temperatur möglich.

Die Neutralisation kann nach der Polymerisation auf der Stufe des Hydrogels durchgeführt werden. Es ist aber auch möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Hydrogels eingestellt wird. Die Monomerlösung kann durch Einmischen des Neutralisationsmittels neutralisiert werden. Das Hydrogel kann mechanisch zerkleinert werden, beispielsweise mittels eines Fleischwolfes, wobei das Neutralisationsmittel aufgesprüht, übergestreut oder aufgegossen und dann sorgfältig untergemischt werden kann. Dazu kann die erhaltene Gelmasse noch mehrmals zur Homogenisierung gewolft werden. Die Neutralisation der Monomerlösung auf den Endneutralisationsgrad ist bevorzugt.

Anschließend werden die erhaltenen wässrigen Hydrogele gemäß den oben beschriebenen erfindungsgemäßen Verfahren getrocknet.

Auf die weitere Behandlung des getrockneten Hydrogels kommt es bei dem erfindungsgemäßen Verfahren nicht an. Das erfindungsgemäße Verfahren kann beispielsweise noch die Schritte Mahlung, Siebung und/oder Nachvernetzung umfassen.

Das getrocknete Hydrogel wird vorzugsweise gemahlen und gesiebt, wobei zur Mahlung üblicherweise Walzenstühle, Stiftmühlen oder Schwingmühlen eingesetzt werden können. Die Partikelgröße des gesiebten, trockenen Hydrogels beträgt vorzugsweise unter 1000 µm, besonders bevorzugt unter 900 µm, ganz besonders bevorzugt unter 800 µm, und vorzugsweise über 100 µm, besonders bevorzugt über 150 µm, ganz besonders bevorzugt über 200 µm.

Ganz besonders bevorzugt ist eine Partikelgröße (Siebschnitt) von 106 bis 850 µm. Die Partikelgröße wird gemäß der von der EDANA (European Disposables and Nonwovens Association) empfohlenen Testmethode Nr. 420.2-02 "Particle size distribution" bestimmt.

Die Grundpolymere werden vorzugsweise anschließend oberflächennachvernetzt. Hierzu geeignete Nachvernetzer sind Verbindungen, die mindestens zwei Gruppen enthalten, die mit den Carboxylatgruppen des Hydrogels kovalente Bindungen bilden können. Geeignete Verbindungen sind beispielsweise Alkoxysiliylverbindungen, Polyaziridine, Polyamine, Polyamidoamine, Di- oder Polyglycidylverbindungen, wie in EP-A-0 083 022, EP-A-543 303 und EP-A-937 736 beschrieben, di- oder polyfunktionelle Alkohole, wie in DE-C-33 14 019, DE-C-35 23 617 und EP-A-450 922 beschrieben, oder ß-Hydroxyalkylamide, wie in DE-A-102 04 938 und US-6,239,230 beschrieben.

Des weiteren sind in DE-A-40 20 780 zyklische Karbonate, in DE-A-198 07 502 2-Oxazolidon und dessen Derivate, wie 2-Hydroxyethyl-2-oxazolidon, in DE-A-198 07 992 Bis- und Poly-2-oxazolidinone, in DE-A-198 54 573 2-Oxotetrahydro-1,3-oxazin und dessen Derivate, in DE-A-198 54 574 N-Acyl-2-Oxazolidone, in DE-A-102 04 937 zyklische Harnstoffe, in DE-A-103 34 584 bizyklische Amidacetale, in EP-A-1 199 327 Oxetane und zyklische Harnstoffe und in WO-A-03/031482 Morpholin-2,3-dion und dessen Derivate als geeignete Oberflächennachvernetzer beschrieben.

Die Nachvernetzung wird üblicherweise so durchgeführt, dass eine Lösung des O-berflächennachvernetzers auf das Hydrogel oder das trockene Grundpolymerpulver aufgesprüht wird. Im Anschluss an das Aufsprühen wird das Polymerpulver thermisch getrocknet, wobei die Vernetzungsreaktion sowohl vor als auch während der Trocknung stattfinden kann.

Das Aufsprühen einer Lösung des Vernetzers wird vorzugsweise in Mischern mit bewegten Mischwerkzeugen, wie Schneckenmischer, Paddelmischer, Scheibenmischer, Pflugscharmischer und Schaufelmischer, durchgeführt werden. Besonders bevorzugt sind Vertikalmischer, ganz besonders bevorzugt sind Pflugscharmischer und Schaufelmischer. Geeignete Mischer sind beispielsweise Lödige®-Mischer, Bepex®-Mischer, Nauta®-Mischer, Processall®-Mischer und Schugi®-Mischer.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Bepex®-Trockner und Nara®-Trockner. Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Es kann aber auch beispielsweise eine azeotrope Destillation als Trocknungsverfahren benutzt werden.

Bevorzugte Trocknungstemperaturen liegen im Bereich 50 bis 250°C, bevorzugt bei 50 bis 200°C, und besonders bevorzugt bei 50 bis 150°C. Die bevorzugte Verweilzeit bei dieser Temperatur im Reaktionsmischer oder Trockner beträgt unter 30 Minuten, besonders bevorzugt unter 10 Minuten.

Das vorliegende Verfahren ermöglicht es, auf einfache Weise wasserabsorbierende Polymere herzustellen, die sich durch ein hervorragendes Absorptionsprofil auszeichnen. Durch das erfindungsgemäße Verfahren wird das bei der Polymerisation anfallende wässrige Polymergel schonend und wirtschaftlich getrocknet. Die Produkteigenschaften werden durch die erfindungsgemäße Trocknung nur wenig beeinflusst. Die auf diese Weise erhaltenen wasserabsorbierenden Polymere können weitestgehend eingesetzt werden in Gebieten, in denen es darum geht, wässrige Flüssigkeiten zu absorbieren und zurückzuhalten. Bevorzugte Einsatzgebiete sind neben dem Hygienesektor vor allem der Agrarsektor sowie weitere industrielle Anwendungsgebiete.

Zur Bestimmung der Güte der Nachvernetzung werden die getrocknete wasserabsorbierenden Polymerpartikel mit den nachfolgend beschrieben Testmethoden geprüft.

Methoden:

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymere werden vor der Messung gut durchmischt.

<u>Zentrifugenretentionskapazität (CRC Centrifuge Retention Capacity)</u>

Die Zentrifugenretentionskapazität der wasserabsorbierenden Polymerpartikel wird gemäß der von der EDANA (European Disposables and Nonwovens Association) empfohlenen Testmethode Nr. 441.2-02 "Centrifuge retention capacity" bestimmt.

<u>Extrahierbare</u>

Der Anteil an Extrahierbaren in den wasserabsorbierenden Polymerpartikeln wird gemäß der von der EDANA (European Disposables and Nonwovens Association) empfohlenen Testmethode Nr. 470.2-02 "Extractables" bestimmt.

<u>Feuchtegehalt:</u>

Der Feuchtegehalt wird gemäß der in der Monographie "Modern Superabsorbent Polymer Technology", F.L. Buchholz und A.T. Graham, Wiley-VCH, 1998, Seiten 143 und 144, beschriebenen Methode bestimmt. Dazu wird 1g Hydrogelprobe in einer LC-Säule unter Heliumathmosphäre 1 Stunde bei 180°C getrocknet und der Feuchtgehalt über den Gewichtsverlust ermittelt.

<u>Trocknungsqualitätsindex (TQI)</u>

Zur Bestimmung des Trocknungsqualitätsindex wird von dem zerkleinerten wässrigen Polymergel nach der Polymerisation eine Probe entnommen, auf Blechen mit Siebböden homogen in dünner Schicht verteilt und dann 24 h bei 80°C im Vakuum bei weniger als 100 mbar getrocknet. Diese Trocknung ist sehr produktschonend. Anschließend wird das getrocknete Hydrogel gemahlen und die Siebfraktion von 300 bis 600 µm isoliert (Polymer 1).

Eine nach dem zu untersuchenden Trockenverfahren getrocknete Hydrogelprobe wird ebenfalls gemahlen. Anschließend wird die Siebfraktion von 300 bis 600 µm isoliert (Polymer 2).

Die getrockneten wasserabsorbierenden Polymere werden durch Bestimmung der Zentrifugenretentionskapazität (CRC) sowie des Gehalts an Extrahierbaren charakterisiert. Zusätzlich wird der Feuchtegehalt bestimmt und rechnerisch bei der Ermittlung dieser Eigenschaften berücksichtigt. Typischerweise liegt der Feuchtegehalt bei ca. 5 Gew.-%.

Aus den Messwerten bestimmt man dann den Trocknungsqualitätsindex (TQI), der sich wie folgt berechnet:

$$TQI = 0{,}5 \times (CRC_2\ [g/g] - CRC_1\ [g/g]) + 0{,}5 \times (Extrahierbare_2\ [\%] - Extrahierbare_1\ [\%])$$

Die tiefgestellten Indizes bezeichnen hier die Polymere 1 bzw. 2. Der Trocknungsqualitätsindex ist also umso größer, je mehr durch die Betriebstrocknung die Zentrifugenretentionskapazität ansteigt und je mehr der Anteil der Extrahierbaren dabei ansteigt. Beide Beiträge werden gleich gewichtet. Die Höhe des Trocknungsqualitätsindex gibt an, wie stark die Eigenschaften des wasserabsorbierenden Polymeren durch die Trocknungsbedingungen des wässrigen Polymergels verändert werden. Ein niediger Trocknungsqualitätsindex bedeutet hierbei eine schonende Trocknung.

Die EDANA-Testmethoden sind beispielsweise erhältlich bei der European Disposables and Nonwovens Association, Avenue Eugene Plasky 157, B-1030 Brüssel, Belgien.

Beispiele:

Herstellung des Polymergels

Durch kontinuierliches Mischen von Wasser, 50 gew.-%iger Natronlauge und Acrylsäure wurde eine 38,8 gew.-%ige Acrylsäure/Natriumacrylatlösung hergestellt, so dass der Neutralisationsgrad 71,3 mol-% betrug. Der Feststoffgehalt der Monomerlösung betrug 38,8 Gew.-%. Die Monomerlösung wurde nach dem Mischen der Komponenten durch einen Wärmetauscher kontinuierlich auf eine Temperatur von 29°C abgekühlt und mit Stickstoff entgast.
Als mehrfach ethylenisch ungesättigter Vernetzer wird Polyethylenglykol-400-diacrylat (Diacrylat eines Polyethylenglykols mit einem mittleren Molgewicht von 400 g/mol) verwendet. Die Einsatzmenge betrug 2 kg pro t Monomerlösung.
Zur Initiierung der radikalischen Polymerisation wurden folgende Komponenten eingesetzt: Wasserstoffperoxid (1,03 kg (0,25 gew.-%ig) pro t Monomerlösung), Natriumperoxodisulfat (3,10 kg (15 gew.-%ig) pro t Monomerlösung), sowie Ascorbinsäure (1,05 kg (1 gew.-%ig) pro t Monomerlösung).
Der Durchsatz der Monomerlösung betrug 18 t/h.
Die einzelnen Komponenten werden kontinuierlich in einen Reaktor List Contikneter mit 6.3m$^3$ Volumen (Fa. List, Arisdorf, Schweiz) in folgenden Mengen eindosiert:

| | |
|---|---|
| 18 t/h | Monomerlösung |
| 36 kg/h | Polyethylenglycol-400-diacrylat |
| 74,34 kg/h | Wasserstoffperoxidlösung/Natriumperoxodisulfat-Lösung |
| 18,9 kg/h | Ascorbinsäurelösung |

Am Ende des Reaktors wurden zusätzlich 750 bis 900 kg/h abgetrenntes Unterkorn mit einer Partikelgröße kleiner 150 $\mu$m zudosiert.
Die Reaktionslösung hatte am Zulauf eine Temperatur von 23,5°C. Der Reaktor wurde mit einer Drehzahl der Wellen von 38rpm betrieben. Die Verweilzeit der Reaktionsmischung im Reaktor betrug 15 Minuten.
Im erhaltenen Produktgel wurde analytisch ein Restacrylsäuregehalt von 0,6 Gew.-% (bezogen auf Feststoffgehalt) und ein Feststoffgehalt von 45,0 Gew.-% gefunden. Eventuell vorhandene Gelpartikel mit einem Durchmesser von 50 mm oder mehr wurden abgetrennt.

Beispiele 1 bis 4 (nicht erfindungsgemäß)

Nach Polymerisation und Gelzerkleinerung wurde das wässrige Polymergel auf einen Bandtrockner aufgegeben. Insgesamt wurden 18,3 t/h wässriges Polymergel mit einem Wassergehalt von 55 Gew.-% getrocknet. Das Gel wurde aus einer Höhe von 30 cm mittels eines Schwenkbandes auf das Förderband des Trockners aufgebracht. Die Höhe der Gelschicht betrug ca. 10 cm.
Die Bandgeschwindigkeit des Trocknerbandes betrug 0,02 m/s und die Verweilzeit auf dem Trocknerband betrug ca. 37 Minuten.
Der Bandtrockner war in insgesamt sechs Temperaturzonen unterteilt, in denen die Gaseingangstemperaturen unabhängig voneinander eingestellt werden konnten. Die Verweilzeit in den Temperaturzonen $T_1$ bis $T_6$ betrug (n=1; a=2; b=3, c=6):

| Temperaturzone | rel. Verweilzeit | abs. Verweilzeit |
|---|---|---|
| T1 | 9% | ca. 3,3 Minuten |
| T2 | 9% | ca. 3,3 Minuten |
| T3 | 14% | ca. 5,2 Minuten |
| T4 | $22^2/_3$% | ca. 8,4 Minuten |
| T5 | $22^2/_3$% | ca. 8,4 Minuten |
| T6 | $22^2/_3$% | ca. 8,4 Minuten |

In den Temperaturzonen $T_1$ bis $T_3$ wurde das Band von unten mit Luft angeströmt. Die Luftgeschwindigkeit betrug 1,2 m/s.

In den Temperaturzonen $T_4$ bis $T_6$ wurde das Band von oben mit Luft angströmt. Die Luftgeschwindigkeit betrug 1,8 m/s.

Die Gaseingangstemperaturen der einzelnen Temperaturzonen sind in der Tabelle 1 und die Messergebnisse sind in der Tabelle 2 zusammengefasst.

Tab. 1: Gaseingangstemperaturen

| Beispiel | $T_1$ | $T_2$ | $T_3$ | $T_4$ | $t_5$ | $T_6$ |
|---|---|---|---|---|---|---|
| 1 | 175°C | 175°C | 175°C | 175°C | 175°C | 175°C |
| 2 | 180°C | 180°C | 175°C | 175°C | 175°C | 175°C |
| 3 | 180°C | 180°C | 165°C | 175°C | 175°C | 175°C |
| 4 | 180°C | 175°C | 165°C | 175°C | 170°C | 165°C |

Tab. 2: Messergebnisse

| Beispiel | $CRC_1$ | $Extrahierbare_1$ | $CRC_2$ | $Extrahierbare_2$ | TQI |
|---|---|---|---|---|---|
| 1 | 34,1 g/g | 8,5 Gew.-% | 44,9 g/g | 16,7 Gew.-% | 9,5 |
| 2 | 34,0 g/g | 8,4 Gew.-% | 42,1 g/g | 14,4 Gew.-% | 7,05 |
| 3 | 34,6 g/g | 8,1 Gew.-% | 39,0 g/g | 12,8 Gew.-% | 4,55 |
| 4 | 34,3 g/g | 8,2 Gew.-% | 34,9 g/g | 8,5 Gew.-% | 0,45 |

Beispiele 5 bis 7 (nicht erfindungsgemäß)

Nach Polymerisation und Gelzerkleinerung wurde das wässrige Polymergel auf einen Bandtrockner aufgegeben. Insgesamt wurden 18,3 t/h wässriges Polymergel mit einem Wassergehalt von 55 Gew.-% getrocknet. Das Gel wurde aus einer Höhe von 30 cm mittels eines Schwenkbandes auf das Förderband des Trockners aufgebracht. Die Höhe der Gelschicht betrug ca. 10 cm.

Die Bandgeschwindigkeit des Trocknerbandes betrug 0,02 m/s und die Verweilzeit auf dem Trocknerband betrug ca. 37 Minuten.

Der Bandtrockner war in insgesamt sechs Temperaturzonen unterteilt, in denen die Gaseingangstemperaturen unabhängig voneinander eingestellt werden konnten. Die Verweilzeit in den Temperaturzonen $T_1$ bis $T_6$ betrug (n=1; a=2; b=3, c=6):

| Temperaturzone | Temperatur | rel. Verweilzeit | abs. Verweilzeit |
|---|---|---|---|
| T1 | 180°C | 9% | ca. 3,3 Minuten |
| T2 | 175°C | 9% | ca. 3,3 Minuten |
| T3 | 165°C | 14% | ca. 5,2 Minuten |
| T4 | 175°C | $22^2/_3$% | ca. 8,4 Minuten |
| T5 | 170°C | $22^2/_3$% | ca. 8,4 Minuten |
| T6 | 165°C | $22^2/_3$% | ca. 8,4 Minuten |

Das Förderband konnte in jeder Temperaturzone unabhängig voneinander wahlweise von unten oder von oben angeströmt werden. Der Wassergehalt bei der Luftumkehr wurde über die Verweilzeit eingestellt. Dazu wurde die Anzahl der von unten bzw. oben angeströmten Temperaturzonen entsprechend variiert.

In den vorderen Temperaturzonen wurde das Band von unten mit Luft angeströmt. Die Luftgeschwindigkeit betrug 1,2 m/s.

In den hinteren Temperaturzone wurde das Band von oben mit Luft angeströmt. Die Luftgeschwindigkeit betrug 1,8 m/s.

Die eingestellten Parameter sind in der Tabelle 3 und die Messergebnisse sind in der Tabelle 4 zusammengefasst.

Tab. 3: Luftgeschwindigkeiten, relative Verweilzeiten und Wassergehalte bei der Strömungsumkehr

| Beispiel | relative Verweilzeit des Hydrogels bei Strömungsumkehr | Wassergehalt des Hydrogels bei Strömungsumkehr |
|---|---|---|
| 5 | 32% | 29,9 Gew.-% |
| 6 | 18% | 36,3 Gew.-% |
| 7 | 54⅓% | 25,8 Gew.-% |

Tab. 4: Messergebnisse

| Beispiel | $CRC_1$ | $Extrahierbare_1$ | $CRC_2$ | $Extrahierbare_2$ | TQI |
|---|---|---|---|---|---|
| 5 | 34,4 g/g | 8,2 Gew.-% | 35,1 g/g | 8,3 Gew.-% | 0,41 |
| 6 | 34,3 g/g | 8,3 Gew.-% | 38,7 g/g | 9,6 Gew.-% | 2,84 |
| 7 | 34,6 g/g | 8,1 Gew.-% | 39,2 g/g | 12,9 Gew.-% | 4,68 |

Beispiele 8 bis 10

Nach Polymerisation und Gelzerkleinerung wurde das wässrige Polymergel auf einen Bandtrockner aufgegeben. Insgesamt wurden 18,3 t/h wässriges Polymergel mit einem Wassergehalt von 55 Gew.-% getrocknet. Das Gel wurde aus einer Höhe von 30 cm mittels eines Schwenkbandes auf das Förderband des Trockners aufgebracht. Die Höhe der Gelschicht betrug ca. 10 cm.

Die für die Trocknung wirksame Förderbandlänge betrug 44 m.

Der Bandtrockner war in insgesamt sechs Temperaturzonen unterteilt, in denen die Gaseingangstemperaturen unabhängig voneinander eingestellt werden konnten. Die Verweilzeit in den Temperaturzonen $T_1$ bis $T_6$ betrug (n=1; a=2; b=3, c=6):

| Temperaturzone | Temperatur | rel. Verweilzeit |
|---|---|---|
| T1 | 180°C | 9% |
| T2 | 175°C | 9% |
| T3 | 165°C | 14% |
| T4 | 175°C | $22^2/_3$% |
| T5 | 170°C | $22^2/_3$% |
| Te | 165°C | $22^2/_3$% |

Die Verweilzeit im Bandtrockner wurde über die Bandgeschwindigkeit eingestellt. Die Gasgeschwindigkeit, bei der sich die Hydrogelschicht vom Band ablöst (Wirbelpunkt), betrug 11 m/s.

In den Temperaturzonen $T_1$ bis $T_3$ wurde das Band von unten mit Luft angeströmt.

In den Temperaturzonen $T_4$ bis $T_6$ wurde das Band von oben mit Luft angeströmt. Die Luftgeschwindigkeit betrug 1,8 m/s.

Die eingestellten Parameter sind in der Tabelle 5 und die Messergebnisse sind in der Tabelle 6 zusammengefasst.

Tab. 5: Luftgeschwindigkeiten, relative Verweilzeiten und Wassergehalte bei der Strömungsumkehr

| Beispiel | Luftgeschwindigkeit vor Strömungsumkehr | relative Luftgeschwindigkeit vor Strömungsumkehr (bezogen auf $v_{max}$) | Verweilzeit im Trockner |
|---|---|---|---|
| 8 | 0,8 m/s | 7% | 50 Minuten |
| 9 | 1,2 m/s | 11% | 30 Minuten |
| 10 | 3,2 m/s | 29% | 40 Minuten |

Tab. 6: Messergebnisse

| Beispiel | Wassergehalt nach Trocknung | TQI |
|---|---|---|
| 8 | 7 Gew.-% | 8,10 |
| 9 | 4 Gew.-% | 0,45 |
| 10*) | 5 Gew.-% | 2,70 |
| *) Rissbildung in Gelschicht | | |

## Patentansprüche

1. Verfahren zur Herstellung wasserabsorbierender Polymere mit niedrigem Trocknungsqualitätsindex durch Polymerisation einer Monomerlösung, enthaltend mindestens eine ethylenisch ungesättigte Carbonsäure und mindestens einen Vernetzer, und Trocknung des erhaltenen Hydrogels mittels eines erwärmten Gasstromes, **dadurch gekennzeichnet, dass**

   - optional die Trocknung in mindestens zwei Temperaturzonen durchgeführt wird, wobei die Gaseingangstemperaturen die Bedingung $T_n$ ungleich $T_{n+a}$ erfüllen, wobei die Indizes n und a jeweils eine ganze Zahl größer 0 bedeuten, die Indizes die zeitliche Abfolge der Temperaturzonen angeben, die das zu trocknende Gut in aufsteigender Abfolge durchläuft und Temperaturzonen mit höheren Indizes später durchlaufen werden, und
   - optional der Gasstrom das Hydrogel im vorderen Abschnitt eines Bandtrockners von unten und im hinteren Abschnitt des Bandtrockners von oben anströmt, wobei die Strömungsumkehr bei einem Wassergehalt des Hydrogels von 15 bis 45 Gew.-% stattfindet, und
   - die Hydrogelschicht in einem Bandtrockner zumindest teilweise von unten angeströmt wird, wobei die Gasgeschwindigkeit von 5 bis 30% der Gasgeschwindigkeit beträgt, die notwendig ist um das Hydrogel vom Band zu lösen, das zu trocknende Hydrogel im vorderen Abschnitt des Bandtrockners von unten und im hinteren Abschnitt des Bandtrockners von oben von dem Gasstrom angeströmt wird und die Gasgeschwindigkeit nach der Strömungsumkehr erhöht ist.

2. Verfahren gemäß Anspruch 1, **dadurch gekennzeichnet, dass** die Gaseingangstemperaturen die Bedingung $T_n$ größer $T_{n+a}$ erfüllen.

3. Verfahren gemäß Anspruch 1 oder 2, **dadurch gekennzeichnet, dass** die Trocknung in mindestens drei Temperaturzonen durchgeführt wird, wobei die Gaseingangstemperaturen die Bedingung $T_{n+a}$ kleiner $T_{n+b}$ erfüllen, wobei der Index b eine ganze Zahl größer a bedeutet.

4. Verfahren gemäß Anspruch 3, **dadurch gekennzeichnet, dass** die Gaseingangstemperaturen die Bedingung $T_n$ größer $T_{n+b}$ erfüllen.

5. Verfahren gemäß einem der Ansprüche 1 bis 4, **dadurch gekennzeichnet, dass** die Gaseingangstemperaturen in mindestens zwei der Temperaturzonen $T_n$ bis $T_{n+a-1}$ die Bedingung $T_{n+r}$ größer $T_{n+s}$ erfüllen, wobei der Index a eine ganze Zahl größer 1, der Index r eine ganze Zahl von 0 bis (a-2) und der Index s eine ganze Zahl von (r+1) bis (a-1) bedeuten.

6. Verfahren gemäß einem der Ansprüche 1 bis 5, **dadurch gekennzeichnet, dass** die Gaseingangstemperaturen in mindestens zwei der Temperaturzonen $T_{n+b}$ bis $T_{n+c-1}$ die Bedingung $T_{n+u}$ größer $T_{n+v}$ erfüllen, wobei der Index c eine ganze Zahl größer (b+1), der Index u eine ganze Zahl von b bis (c-2) und der Index v eine ganze Zahl von (u+1) bis (c-1) bedeuten.

7. Verfahren gemäß einem der Ansprüche 1 bis 6, **dadurch gekennzeichnet, dass** die Gaseingangstemperaturen in mindestens drei der Temperaturzonen $T_{n+b}$ bis $T_{n+c-1}$ die Bedingung $T_{n+u}$ größer $T_{n+v}$ größer $T_{n+w}$ erfüllen, wobei der Index c eine ganze Zahl größer (b+2), der Index u eine ganze Zahl von b bis (c-3), der Index v eine ganze Zahl von (u+1) bis (c-2) und der Index w eine ganze Zahl von (v+1) bis (c-1) bedeuten.

8. Verfahren gemäß einem der Ansprüche 1 bis 7, **dadurch gekennzeichnet, dass** die Temperaturdifferenz der

Gaseingangstemperaturen mindestens 2°C beträgt.

9. Verfahren gemäß einem der Ansprüche 1 bis 8, **dadurch gekennzeichnet, dass** der Gasstrom das Hydrogel im vorderen Abschnitt des Bandtrockners von unten und im hinteren Abschnitt des Bandtrockners von oben anströmt, wobei die Strömungsumkehr bei einem Wassergehalt des Hydrogels von 20 bis 40 Gew.-% stattfindet.

10. Verfahren gemäß einem der Ansprüche 1 bis 9, **dadurch gekennzeichnet, dass** die Hydrogelschicht zumindest teilweise von unten angeströmt wird, wobei die Gasgeschwindigkeit von 10 bis 20% der Gasgeschwindigkeit beträgt, die notwendig ist um das Hydrogel vom Band zu lösen.

11. Verfahren gemäß einem der Ansprüche 1 bis 10, **dadurch gekennzeichnet, dass** das Hydrogel vor der Trocknung einen Wassergehalt von 30 bis 70 Gew.-% aufweist.

12. Verfahren gemäß einem der Ansprüche 1 bis 11, **dadurch gekennzeichnet, dass** das Hydrogel nach der Trocknung einen Wassergehalt von 1 bis 10 Gew.-% aufweist.

13. Verfahren gemäß einem der Ansprüche 1 bis 12, **dadurch gekennzeichnet, dass** die Geschwindigkeit des die Hydrogelschicht anströmenden Gasstroms von 0,5 bis 5 m/s beträgt.

14. Verfahren gemäß einem der Ansprüche 1 bis 13, **dadurch gekennzeichnet, dass** der die Hydrogelschicht anströmende Gasstrom einen Wasserdampfanteil aufweist, der einem Taupunkt von höchstens 50°C entspricht.

15. Verfahren gemäß einem der Ansprüche 1 bis 14, **dadurch gekennzeichnet, dass** die Temperatur des die Hydrogelschicht anströmenden Gasstroms von 50 bis 250°C beträgt.

16. Verfahren gemäß einem der Ansprüche 1 bis 15, **dadurch gekennzeichnet, dass** die Verweilzeit des Hydrogels im Trockner von 10 bis 120 Minuten beträgt.

17. Verfahren gemäß einem der Ansprüche 1 bis 16, **dadurch gekennzeichnet, dass** der Druck bei der Trocknung niedriger ist als Atmosphärendruck.

18. Verfahren gemäß einem der Ansprüche 1 bis 17, **dadurch gekennzeichnet, dass** die Aufgabehöhe des Hydrogels auf das Band von 10 bis 200 cm beträgt.

19. Verfahren gemäß einem der Ansprüche 1 bis 18, **dadurch gekennzeichnet, dass** die Bandgeschwindigkeit von 0,005 bis 0,05 m/s beträgt.

## Claims

1. A process for producing a water-absorbing polymer having a low Drying Quality Index by polymerizing a monomer solution comprising at least one ethylenically unsaturated carboxylic acid and at least one crosslinker and drying the resulting hydrogel by means of a heated gas stream, which comprises

   - optionally effecting the drying in two or more temperature zones for which the gas inlet temperatures satisfy the condition $T_n$ unequal $T_{n+a}$, where the indices n and a are each a whole number greater than 0, the indices indicate the chronological order of the temperature zones which the dryer feedstock traverses in ascending order in that temperature zones having higher indices are traversed later, and
   - optionally the gas stream is flowed against the hydrogel upwardly in the upstream sector of a belt dryer and downwardly in the downstream sector of the belt dryer, the direction of flow being reversed at a water content of 15% to 45% by weight for the hydrogel, and
   - the hydrogel layer is flowed against in a belt dryer upwardly to some extent at least, the gas velocity being 5% to 30% of the gas velocity required to lift the hydrogel off the belt, the hydrogel which is to be dried being flowed against by the gas stream upwardly in the upstream sector of the belt dryer and downwardly in the downstream sector of the belt dryer and the gas velocity being in an elevated state after the reversal of the direction of flow.

2. The process according to claim 1 wherein the gas inlet temperatures satisfy the condition of $T_n$ greater than $T_{n+a}$.

**3.** The process according to claim 1 or 2 wherein the drying is effected in three or more temperature zones for which the gas inlet temperatures satisfy the condition of $T_{n+a}$ less than $T_{n+b}$, where the index b is a whole number greater than a.

**4.** The process according to claim 3 wherein the gas inlet temperatures satisfy the condition of $T_n$ greater than $T_{n+b}$.

**5.** The process according to any one of claims 1 to 4 wherein the gas inlet temperatures in two or more of the temperatures zones $T_n$ to $T_{n+a-1}$ satisfy the condition $T_{n+r}$ greater than $T_{n+s}$, where the index a is a whole number greater than 1, the index r is a whole number from 0 to (a-2) and the index s is a whole number from (r+1) to (a-1).

**6.** The process according to any one of claims 1 to 5 wherein the gas inlet temperatures in two or more of the temperature zones $T_{n+b}$ to $T_{n+c-1}$ satisfy the condition of $T_{n+u}$ greater than $T_{n+v}$, where the index c is a whole number greater than (b+1), the index u is a whole number from b to (c-2) and the index v is a whole number from (u+1) to (c-1).

**7.** The process according to any one of claims 1 to 6 wherein the gas inlet temperatures in three or more of the temperature zones $T_{n+b}$ to $T_{n+c-1}$ satisfy the condition of $T_{n+u}$ greater than $T_{n+v}$ greater than $T_{n+w}$, where the index c is a whole number greater than (b+2), the index u is a whole number from b to (c-3), the index v is a whole number from (u+1) to (c-2) and the index w is a whole number from (v+1) to (c-1) .

**8.** The process according to any one of claims 1 to 7 wherein the gas inlet temperatures differ by not less than 2°C.

**9.** The process according to any one of claims 1 to 8 wherein the gas stream is flowed against the hydrogel upwardly in the upstream sector of the belt dryer and downwardly in the downstream sector of the belt dryer, the direction of flow being reversed at a water content of 20% to 40% by weight for the hydrogel.

**10.** The process according to any one of claims 1 to 9 wherein the hydrogel layer is flowed against in a belt dryer upwardly to some extent at least, the gas velocity being 10% to 20% of the gas velocity required to lift the hydrogel off the belt.

**11.** The process according to any one of claims 1 to 10 wherein the hydrogel has a water content in the range from 30% to 70% by weight before drying.

**12.** The process according to any one of claims 1 to 11 wherein the hydrogel has a water content in the range from 1% to 10% by weight after drying.

**13.** The process according to any one of claims 1 to 12 wherein the velocity of the gas stream flowing against the hydrogel layer is in the range from 0.5 to 5 m/s.

**14.** The process according to any one of claims 1 to 13 wherein the gas stream flowing against the hydrogel layer has a water vapor content which corresponds to a dew point of not more than 50°C.

**15.** The process according to any one of claims 1 to 14 wherein the temperature of the gas stream flowing against the hydrogel layer is in the range from 50 to 250°C.

**16.** The process according to any one of claims 1 to 15 wherein the residence time of the hydrogel in the dryer is in the range from 10 to 120 minutes.

**17.** The process according to any one of claims 1 to 16 wherein the pressure prevailing during the drying is lower than atmospheric pressure.

**18.** The process according to any one of claims 1 to 17 wherein the feed height of the hydrogel onto the belt is in the range from 10 to 200 cm.

**19.** The process according to any one of claims 1 to 18 wherein the belt speed is in the range from 0.005 to 0.05 m/s.

## Revendications

1. Procédé pour la préparation de polymères absorbant de l'eau avec un indice de qualité de séchage bas, par polymérisation d'une solution de monomères contenant au moins un acide carboxylique éthyléniquement insaturé et au moins un réticulant, et séchage de l'hydrogel obtenu au moyen d'un flux gazeux chauffé, **caractérisé en ce que**

   - le séchage est éventuellement réalisé dans au moins deux zones de température, où les températures d'entrée du gaz satisfont à la condition $T_n$ différent de $T_{n+a}$, où les indices n et a valent chacun un nombre entier supérieur à 0, les indices indiquent la séquence temporelle des zones de température à travers lesquelles passe le produit à sécher en succession croissante, les zones de température d'indices plus élevés étant parcourues plus tard, et
   - le flux gazeux s'écoule éventuellement dans l'hydrogel dans la section antérieure d'un sécheur à bande à partir du bas et dans la section postérieure du sécheur à bande à partir du haut, où l'inversion du flux a lieu à une teneur en eau de l'hydrogel de 15 à 45% en poids, et
   - la couche d'hydrogel est soumise à un flux gazeux dans un sécheur à bande au moins partiellement à partir du bas, où la vitesse du gaz est de 5 à 30% de la vitesse du gaz qui est nécessaire pour détacher l'hydrogel de la bande, l'hydrogel à sécher est soumis au flux gazeux dans la section antérieure du sécheur à bande à partir du bas et dans la section postérieure du sécheur à bande à partir du haut et la vitesse du gaz est augmentée après l'inversion du flux.

2. Procédé selon la revendication 1, **caractérisé en ce que** les températures d'entrée du gaz satisfont à la condition $T_n$ supérieur à $T_{n+a}$.

3. Procédé selon la revendication 1 ou 2, **caractérisé en ce que** le séchage est réalisé dans au moins trois zones de température, où les températures d'entrée du gaz satisfont à la condition $T_{n+a}$ inférieur à $T_{n+b}$, où l'indice b signifie un nombre entier supérieur à a.

4. Procédé selon la revendication 3, **caractérisé en ce que** les températures d'entrée du gaz satisfont à la condition $T_n$ supérieur à $T_{n+b}$.

5. Procédé selon l'une quelconque des revendications 1 à 4, **caractérisé en ce que** les températures d'entrée du gaz satisfont dans au moins deux des zones de température $T_n$ à $T_{n+a-1}$ à la condition $T_{n+r}$ supérieur à $T_{n+s}$, où l'indice a signifie un nombre entier supérieur à 1, l'indice r signifie un nombre entier de 0 à (a-2) et l'indice s signifie un nombre entier de (r+1) à (a-1) .

6. Procédé selon l'une quelconque des revendications 1 à 5, **caractérisé en ce que** les températures d'entrée du gaz satisfont dans au moins deux des zones de température $T_{n+b}$ à $T_{n+c-1}$ à la condition $T_{n+u}$ supérieur à $T_{n+v}$, où l'indice c signifie un nombre entier supérieur à (b+1), l'indice u signifie un nombre entier de b à (c-2) et l'indice v signifie un nombre entier de (u+1) à (c-1) .

7. Procédé selon l'une quelconque des revendications 1 à 6, **caractérisé en ce que** les températures d'entrée du gaz satisfont dans au moins trois des zones de température $T_{n+b}$ à $T_{n+c-1}$ à la condition $T_{n+u}$ supérieur à $T_{n+w}$, où l'indice c signifie un nombre entier supérieur à (b+2), l'indice u signifie un nombre entier de b à (c-3) et l'indice v signifie un nombre entier de (u+1) à (c-2) et l'indice w signifie un nombre entier de (v+1) à (c-1).

8. Procédé selon l'une quelconque des revendications 1 à 7, **caractérisé en ce que** la différence des températures d'entrée du gaz est d'au moins 2°C.

9. Procédé selon l'une quelconque des revendications 1 à 8, **caractérisé en ce que** le flux gazeux s'écoule dans l'hydrogel dans la section antérieure du sécheur à bande à partir du bas et dans la section postérieure du sécheur à bande à partir du haut, où l'inversion du flux a lieu à une teneur en eau de l'hydrogel de 20 à 40% en poids.

10. Procédé selon l'une quelconque des revendications 1 à 9, **caractérisé en ce que** la couche d'hydrogel est soumise à un flux gazeux au moins partiellement à partir du bas, où la vitesse du gaz est de 10 à 20% de la vitesse du gaz qui est nécessaire pour détacher l'hydrogel de la bande.

11. Procédé selon l'une quelconque des revendications 1 à 10, **caractérisé en ce que** l'hydrogel présente, avant le séchage, une teneur en eau de 30 à 70% en poids.

**12.** Procédé selon l'une quelconque des revendications 1 à 11, **caractérisé en ce que** l'hydrogel présente, après le séchage, une teneur en eau de 1 à 10% en poids.

**13.** Procédé selon l'une quelconque des revendications 1 à 12, **caractérisé en ce que** la vitesse du flux gazeux s'écoulant dans la couche d'hydrogel est de 0,5 à 5 m/s.

**14.** Procédé selon l'une quelconque des revendications 1 à 13, **caractérisé en ce que** le flux gazeux s'écoulant dans la couche d'hydrogel présente une proportion de vapeur d'eau qui correspond à un point de rosée d'au maximum 50°C.

**15.** Procédé selon l'une quelconque des revendications 1 à 14, **caractérisé en ce que** la température du flux gazeux s'écoulant dans la couche d'hydrogel est de 50 à 250°C.

**16.** Procédé selon l'une quelconque des revendications 1 à 15, **caractérisé en ce que** le temps de séjour de l'hydrogel dans le sécheur est de 10 à 120 minutes.

**17.** Procédé selon l'une quelconque des revendications 1 à 16, **caractérisé en ce que** la pression lors du séchage est inférieure à la pression atmosphérique.

**18.** Procédé selon l'une quelconque des revendications 1 à 17, **caractérisé en ce que** la hauteur d'alimentation de l'hydrogel sur la bande est de 10 à 200 cm.

**19.** Procédé selon l'une quelconque des revendications 1 à 18, **caractérisé en ce que** la vitesse de la bande est de 0,005 à 0,05 m/s.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 19846413 A1
- EP 0785224 A
- EP 0785223 A
- DE 19846413 A
- EP 0530438 A
- EP 0547847 A
- EP 0559476 A
- EP 0632068 A
- WO 9321237 A
- WO 03104299 A
- WO 03104300 A
- WO 03104301 A
- DE 10331450 A
- DE 10331456 A
- DE 10355401
- DE 19543368 A
- DE 19646484 A
- WO 9015830 A
- WO 0232962 A
- EP 0343427 A
- DE 19941423 A
- EP 0686650 A
- WO 0145758 A
- EP 0445619 A
- WO 0138402 A
- DE 3825366 A
- US 6241928 B
- EP 0457660 A
- WO 0294328 A
- WO 0294329 A
- EP 0955086 A
- EP 0083022 A
- EP 543303 A
- EP 937736 A
- DE 3314019 C
- DE 3523617 C
- EP 450922 A
- DE 10204938 A
- US 6239230 B
- DE 4020780 A
- DE 198075022 A
- DE 19807992 A
- DE 198545732 A
- DE 10204937 A
- DE 10334584 A
- EP 1199327 A
- WO 03031482 A

### In der Beschreibung aufgeführte Nicht-Patentliteratur

- **F.L. BUCHHOLZ ; A.T. GRAHAM.** Modern Superabsorbent Polymer Technology. Wiley-VCH, 1998
- Ullmann's Encyclopedia of Industrial Chemistry. vol. 35, 73-103
- **F.L. BUCHHOLZ ; A.T. GRAHAM.** Modern Superabsorbent Polymer Technology. Wiley-VCH, 1998, 87-93
- **W.C. GRIFFIN.** *J.Soc.Cosmetic Chem.,* 1954, vol. 5, 249
- **F.L. BUCHHOLZ ; A.T. GRAHAM.** Modern Superabsorbent Polymer Technology. Wiley-VCH, 1998, 143-144